(19) Europäisches Patentamt
European Patent Office
Office européen des brevets

(11) **EP 2 267 168 B1**

(12) # EUROPEAN PATENT SPECIFICATION

(45) Date of publication and mention of the grant of the patent:
**26.07.2017 Bulletin 2017/30**

(21) Application number: **08734017.0**

(22) Date of filing: **23.04.2008**

(51) Int Cl.:
***C12Q 1/02*** *(2006.01)* ***C12M 1/36*** *(2006.01)*
***C12M 1/34*** *(2006.01)* ***G01N 33/50*** *(2006.01)*
***G01N 33/569*** *(2006.01)*

(86) International application number:
**PCT/CN2008/000830**

(87) International publication number:
**WO 2009/129654 (29.10.2009 Gazette 2009/44)**

(54) **METHOD AND DEVICE FOR CONTROLLING FERMENTING PROCESS**

VERFAHREN UND VORRICHTUNG ZUR STEUERUNG EINES FERMENTATIONSPROZESSES

PROCÉDÉ ET DISPOSITIF DE RÉGULATION DE PROCESSUS DE FERMENTATION

(84) Designated Contracting States:
**AT BE BG CH CY CZ DE DK EE ES FI FR GB GR HR HU IE IS IT LI LT LU LV MC MT NL NO PL PT RO SE SI SK TR**

(43) Date of publication of application:
**29.12.2010 Bulletin 2010/52**

(73) Proprietor: **Siemens Aktiengesellschaft**
**80333 München (DE)**

(72) Inventors:
- **FAN, Shun Jie**
  **Beijing 100096 (CN)**
- **YANG, Hongwei**
  **76187 Karlsruhe (DE)**
- **JIANG, Jun Feng**
  **Beijing 100028 (CN)**
- **HERBERT, Grieb**
  **76316 Malsch (DE)**
- **ZHUO, Yue**
  **Beijing 100084 (CN)**
- **KURT, Bettenhausen**
  **65474 Bischofsheim (DE)**

(56) References cited:
**EP-A1- 1 728 853** **EP-A2- 0 661 380**
**WO-A2-02/083836** **US-A- 4 856 421**
**US-A- 5 629 202**

- **RUI ZHAO ET AL: "A flow injection flow cytometry system for on-line monitoring of bioreactors", BIOTECHNOLOGY AND BIOENGINEERING, vol. 62, no. 5, 5 March 1999 (1999-03-05), pages 609-617, XP055071525, ISSN: 0006-3592, DOI: 10.1002/(SICI)1097-0290(19990305)62:5<609: :AID-BIT13>3.0.CO;2-C**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 267 168 B1

## Description

Field of the Invention

The present invention relates to bioengineering technology, and more particularly to a method and device for controlling the fermentation process.

Background of the Invention

The biological fermentation process is a key technique in various industrial fields, e.g. biopharmaceuticals, biological energy, foods and beverages, and environmental protection. An important goal of the biological fermentation process is to improve the consistency of the production process. However, due to the time-dependent change and non-linear properties of the biological fermentation process and the effect of other indeterminable factors, there are often considerable variations among different fermentation batches. How to control the biological fermentation process to deliver high consistency so as to improve the fermentation quality has become an important direction for research.

The biological fermentation process in the present invention includes biological fermentation and cell culture. Industrial biological fermentation processes normally take place in a fermenting tank in which nutrient solution is added and suitable environmental conditions are maintained so that biological cells grow and reproduce in the nutrient solution and produce desired products. The operator or automatic control system can control the biological fermentation process by adjusting the feed rate of the nutrient solution flow to the fermenting tank. In the prior art, the open-loop control of the fermentation process is achieved by means of monitoring and analyzing fermenting environment parameters, such as temperature and pH value of the nutrient solution and concentration of dissolved oxygen, and following the predetermined nutrient solution feeding strategy. However, these control methods only involve external factors like fermenting environment, but ignore the status changes of biological cells, especially the effect of status changes such as cell size, shape, and structure, on the fermentation process. As the status changes of biological cells during the fermentation process imposes considerable effects on the fermentation process, it is difficult for these control methods in the prior art to ensure the consistency of the fermentation process.

The WO 02/083836 A2 discloses a method of controlling the feed rate of nutrient into a fermentor comprising: (a) setting a target concentration of nutrient in said fermentor; (b) determining future demand for nutrient in said fermentor based on a nutrient mass balance equation, wherein said nutrient mass balance equation uses measured, approximated and calculated data, thereby determining the nutrient feed rate into said fermentor.

Rui Zhao ET AL: "A flow injection flow cytometry system for on-line monitoring of bioreactors", Biotechnology and Bioengineering, vol. 62, no. 5, 5 March 1999 (1999-03-05), pages 609-617 describes a flow injection flow cytometry system for online monitoring of bioreactors and online single assessment of single cell light scattering distributions.

Summary of the Invention

In view of the aforesaid problems, the object of the present invention is to provide a method and device for controlling the fermentation process to achieve control of the bio-fermentation process according to the status information of the biological cells, thus improving the consistency of the fermentation process.

The invention provides a method for controlling the fermentation process, wherein the method comprises:

A. collecting samples of biological cells with a flow cytometer from the fermenting tank and outputting a scatter plot compromising the current status information of the biological cells according to the biological cell samples, said status information comprising any one or any combination of cell concentration information, cell size information, cell form information, cell structure information and cell metabolism information;

B. comparing said scatter plot of current status information of the biological cells with the scatter plot of the preset target status information to obtain the difference status between the current status information of the biological cells and the target status information, whereby said obtaining the difference status comprises:

> determining the center of gravity of the scatter plot of the current status information of the biological cells and the center of gravity of the scatter plot of said preset target status information;
> determining the vector characterizing said difference status, wherein the vector starts from the center of gravity of the scatter plot of the current status information of the biological cells, and points to and ends at the center of gravity of the scatter plot of said preset target status information;

C. controlling the feed rate of the nutrient solution flow to said fermenting tank according to said obtained difference status.

The invention further provides a device for controlling the fermentation process, comprising a flow cytometer, a comparison unit and a control unit;
said flow cytometer comprising: a collection subunit, a flow chamber, a laser generator and a detector;

said collection subunit is used to collect biological cell samples from the fermenting tank and inject the biological cell samples into the center of the sheath fluid in a flow chamber;
said flow chamber is used to allow the biological cells in said samples to align in a single line and form a cell bundle under the restriction of the sheath fluid;
said laser generator is used to generate a laser and excite said cell bundle to produce light information;
said detector is used to obtain the current status information of the biological cells from said light information according to the biological cell samples, said status information comprising any one or any combination of cell concentration information, cell size information, cell form information, cell structure information and cell metabolism information;
said comparison unit is used to compare said current status information of the biological cells with the preset target status information, obtain the difference status between the current status information of the biological cells and the preset target status information, and provide the difference status to said control unit, whereby said obtaining the difference status comprises:

determining the center of gravity of the scatter plot of the current status information of the biological cells and the center of gravity of the scatter plot of said preset target status information;
determining the vector characterizing said difference status, wherein the vector starts from the center of gravity of the scatter plot of the current status information of the biological cells, and points to and ends at the center of gravity of the scatter plot of said preset target status information; said control unit is used to control the feed rate of nutrient solution flow to said fermenting tank according to said difference status.

As shown in the aforesaid technical solution, the method and device provided by the present invention comprise: collecting samples of biological cells from the fermenting tank and obtaining current status information of biological cells according to the samples; comparing current status information of biological cells with preset target status information to obtain the difference between current status information of biological cells and target status information; controlling the feed rate of nutrient solution flow to the fermenting tank according to the obtained difference status. In this way, the biological fermentation process can be controlled according to the status information of the biological cells during the fermentation process, thereby improving the consistency during the fermentation process.

In addition, the method and device do not require complicated data analysis and can achieve real-time control of the biological fermentation process through a closed feedback loop, does not have control delay while it achieves automatic control of cell status during the fermentation process.

Brief Description of the Drawings

Fig.1 is a flow chart of the method provided by the embodiment according to the present invention;

Fig. 2 is the current FALS-RALS scatter plot of biological cells provided by the embodiment according to the present invention;

Fig. 3 is a schematic diagram showing acquisition of status difference information of biological cells provided by the embodiment according to the present invention;

Fig. 4 is a schematic diagram showing that the determined feed rate of nutrient solution flow is within the preset range of flow feed rate provided by the embodiment according to the present invention;

Fig. 5 shows the structure of the device provided by the embodiment according to the invention;

Fig. 6 is a schematic diagram showing the structure of the flow cytometer provided by the embodiment according to the present invention.

Exemplary Embodiments

In order to clarify the purposes, technical solution and benefits of the present invention, the following further describe the present invention in combination with drawings and embodiments.

The method provided by the present invention mainly comprises: collecting biological cell samples from the fermenting tank and obtaining current status information of biological cells according to the biological cell samples; comparing the current status information of biological cells with the preset target status information to obtain the difference status between the current status information of biological cells and the target status information; controlling the feed rate of nutrient solution flow to the fermenting tank according to the obtained difference status.

As shown in Fig.1, the method may comprise the following steps:

Step 101: collecting biological cell samples from the fermenting tank and obtaining the current status information of the biological cells according to the biological cell samples.

In this step, real-time collection of biological cell samples and determination of status information of biological cells can be achieved by using a biological cell status determination instrument, such as a flow cytometer (FCM). Said FCM injects the collected biological cell samples into the center of the sheath fluid, and causes the biological cells to align in a single line and form a cell bundle under the restriction of sheath fluid; after being excited by laser, said cell bundle produces a scattering light, of which the forward angle light scattering (FALS) and right angle light scattering (RALS) are collected and undergo photoelectric signal conversion; the electrical pulse signals obtained from FALS and RALS undergoing photoelectric signal conversion form a FALS-RALS scatter plot, from which cell concentration information, cell size information and cell form information can be obtained. The FALS-RALS scatter plot may be as shown in Fig. 2, wherein the x axis indicates the electrical FALS pulse signals and the y axis indicates the electrical RALS pulse signals. In addition, cells may also be first dyed with a fluorescent dye, and the waveband of the scattered light produced by the laser-excited cell bundle is detected, and the obtained waveband information is used to form a scatter plot of waveband information, from which cell structure information and cell metabolism information can be obtained. In this way, the present invention is not limited to the method for acquiring specific cell status information.

Step 102: comparing the current status information of biological cells with the preset target status information to obtain the difference status between the current status information of biological cells and the preset target status information.

If a FALS-RALS scatter plot is obtained from FCM, the preset target status information may be the optimal FALS-RALS scatter plot obtained from previous fermentation batches, or may be the optimal FALS-RALS scatter plot obtained from experiments. The optimal FALS-RALS scatter plot may be the FALS-RALS scatter plot that reflects the highest cell concentration and the best product expression rate.

The difference status between the current status information of biological cells and the target status information may be expressed by a vector $\bar{e}$. Taking the FALS-RALS scatter plot as example, Fig. 3 is a schematic diagram showing the steps for obtaining the vector, $\bar{e}$, wherein the scatter plot of target status information and the scatter plot of current status information of biological cells are displayed in the same FALS-RALS coordinate system. The center of gravity of the scatter plot for target status information is determined as the center of gravity 1; the center of gravity of the scatter plot for current status information of biological cells is determined as the center of gravity 2. The vector $\bar{e}$ starts from the center of gravity of the scatter plot for current status information of biological cells, and points to and ends at the center of gravity of the scatter plot for target status information, i.e., vector $\bar{e}$ starts from the center of gravity 2 and points to and ends at the center of gravity 1. The vector $\bar{e}$ can be expressed as:

$$\vec{e}=[e_m,e_x,e_y]^T 。$$

In this case, $e_m$ indicates the density difference between the scatter plot for current status information of biological cells and the scatter plot for target status information, characterizing the difference between cell concentration during current fermentation process and target cell concentration; $e_x$ indicates the difference between the center of gravity 2 and the center of gravity 1, characterizing the difference in size between the cells, during the current fermentation process and the target cell size; $e_y$ indicates the difference between the center of gravity 1 and the center of gravity 2 in the y axis, characterizing the difference in form between the cell form during current fermentation process and the target cell form. Therefore, the vector $\bar{e}$ can be used to characterize the difference in cell concentration, cell size and cell form between the status information during current fermentation process and the target status information.

Step 103: determining feed rate of nutrient solution into the fermenting tank according to the obtained difference status information.

In this step, in order to utilize the difference status in a more straightforward and direct way, the vector $\bar{e}$ may be first converted to a scalar according to the preset conversion strategy. The conversion strategy may be linear conversion, non-linear conversion, etc.

The converted scalar e may be expressed as $e=f(\bar{e})$, wherein f is the conversion strategy adopted. Here, taking linear conversion as the example, the converted scalar e may be expressed as:

$$e=[k_1,k_2,k_3]\,\vec{e}.$$

In this case, $0 \le k_1,k_2,k_3 \le 1$. $k_1,k_2,k_3$ may indicate the weights of cell concentration, cell size and cell form in the control process respectively, and can be set according to specific control requirements. For example, if the only purpose of fermentation process control is to allow the cell concentration to approach as close as possible the target cell concentration, then $k_2$ and $k_3$ may be set as 0 and $k_1$ may be set as 1.

After the scalar e is obtained from the conversion, the feed rate of nutrient solution flow to the fermenting tank is determined according to said scalar e. The determination process is conducted according to the predetermined flow feed rate determination strategy so that the determined feed rate of nutrient solution flow can minimize the difference between the status information of biological cells during fermentation process and the target status, i.e. the status information of biological cells in the fermenting tank converges to the target status information. The specific flow feed rate determination strategies can be in various forms, e.g. proportional-integral-derivative (PID) strategy, model predictive control strategy, fuzzy control strategy and neural network control strategy, etc. Here, taking PID strategy as the example, the feed rate F(t) of nutrient solution flow to the fermenting tank may be expressed as:

$$F(t) = F_p(t) + K_C[e(t) + \frac{1}{\tau_I}\int_0^t e(t)dt + \tau_D \frac{de(t)}{dt}]$$

In this case, $F_p(t)$ indicates the preset target feed rate of nutrient solution, and proportionality constant $K_c$ and integration constant $\tau_1$ may be determined by experimental paradigms according to the dynamic response of biological cells in the fermenting tank. For example, $K_c$ and $\tau_1$ that minimize the scalar e may be determined by the dynamic response information of biological cells in the fermenting tank during experiments. As the measured noise of the biological attribute is large, integration may be neglected, i.e. $\tau_D$ may be set to 0 and the constants in F(t) should be selected in a way to minimize the scalar e.

The feed rate of nutrient solution flow determined at this point may be used as the feed rate of nutrient solution flow to the fermenting tank and the process is now finished. Preferably, the following step may be further performed:

Step 104: judging whether the determined feed rate of nutrient solution flow is within the preset range of feed rate; if yes, proceed to step 105; if no, proceed to step 106.

As various noises or device failures in the control device may cause considerable error in the determined nutrient feed rate, in order to prevent such considerable error from affecting the fermentation process control, the flow feed rate range can be preset. The flow feed rate range is the acceptable range of feed rates of nutrient solution flow. If the determined feed rate of nutrient solution flow is within said flow feed rate range, as shown in Fig. 4, it indicates that there is no considerable error and the determined feed rate of nutrient solution flow can be directly used as the feed rate of nutrient solution flow to the fermenting tank; if the determined feed rate of nutrient solution flow is outside said flow feed rate range, it indicates that there is considerable error and the preset target feed rate of nutrient solution flow is directly used as the feed rate of nutrient solution flow to the fermenting tank, as shown in steps 105 and 106 below.

Step 105: using the determined feed rate of nutrient solution flow as the feed rate of nutrient solution flow to the fermenting tank and ending the process.

Step 106: using the preset target feed rate of nutrient solution flow as the feed rate of nutrient solution flow to the fermenting tank.

As various steps in the aforesaid process take place during the fermentation process, and the step 101 is executed again following step 106, a closed-loop feedback control process is formed.

The above is the detailed description of the method provided by the present invention, and the device provided by the present invention is described below. Fig. 5 shows the structural diagram of the device provided by the embodiment according to the present invention. As shown in Fig. 5, the device may comprise: a status information acquisition unit 500, a comparison unit 510 and a control unit 520.

The status information acquisition unit 500 is used to collect samples of biological cells from the fermenting tank and obtain the status information of the current biological cells according to the biological cell samples.

The comparison unit 510 is used to compare the current status information of said biological cells with preset target status information, obtain the difference status between the current status information of the biological cells and preset target status information, and provide the difference status to the control unit 520.

The control unit 520 is used to control the feed rate of nutrient solution into the fermenting tank according to the difference status.

In this case, said status information acquisition unit 500 may be a flow cytometer and the specific structure is shown in Fig. 6. Said flow cytometer may comprise: a collection subunit 601, a flow chamber 602, a laser generator 603 and a detector 604.

The collection subunit 601 is used to collect samples of biological cells from fermenting tank and inject said samples into the center of sheath fluid in flow chamber 602.

The flow chamber 602 is used to allow biological cells in said samples to align in a single line and form a cell bundle under the restriction of sheath fluid.

The laser generator 603 is used to generate a laser and excite said cell bundle to produce light information.

The detector 604 is used to obtain the current status information of biological cells from said light information.

The detector 604 may be a photodiode or a photomultiplier tube, which collects scattered light or fluorescence produced by laser-excited cells and converts light into electrical signals. The converted electrical signals can be used to form the status information scatter plot. For example, FALS and RALS scattered light can be collected and converted to obtain a FALS-RALS scatter plot and scattered light may be detected for waveband to obtain a waveband information scatter plot; cell concentration information, cell size information and cell form information can be obtained from the FALS-RALS scatter plot, and cell structure information and cell metabolism information can be obtained from waveband information scatter plot. The detector 1 in detector 604 shown in Fig. 6 can collect FALS scattered light and convert light into electrical signals, and detector 2 can collect RALS scattered light and convert light into electrical signals.

The comparison unit 510 can use the method in method step 102 to conduct comparison and obtain the difference status. Taking the FALS-RALS scatter plot, the comparison unit 510 can firstly determine the center of gravity of the scatter plot for target status information, expressed as center of gravity 1, and the center of gravity of the scatter plot for current status information of biological cells, expressed as center of gravity 2, and take the vector $\overline{e}$ starting from the center of gravity 2 and pointing to and ending at the center of gravity 1 to indicate the difference between the current status information of biological cells and target status information, and then provide the vector $\overline{e}$ to the control unit 520. The control unit 520 can firstly convert the vector $\overline{e}$ to a scalar according to a predetermined conversion strategy, and then take the converted scalar e as a variable to determine the nutrient feed rate according to a predetermined flow feed rate determination strategy that allows the status information of biological cells in the fermenting tank to converge to target status information, wherein, the feed rate determination strategy includes various modes, such as PID strategy.

Moreover, said device may also comprise: a target status information storage unit 530 for storing target status information.

The target status information storage unit 530 can store target status information that corresponds to different application needs; an operator can select target status information suited to the current fermentation process according to different application needs for the comparison unit 510 to conduct comparison, and can update target status information at any time as needed.

The comparison unit 510 is also used to obtain target status information from the target status information storage unit 530.

In this case, said control unit 520 may comprise: a flow feed rate determination subunit 521 and a feeding operation subunit 522.

The flow feed rate determination subunit 521 is used to determine the feed rate of nutrient solution flow according to the difference status by using the predetermined flow feed rate determination strategy which causes the status information of biological cells in the fermenting tank to converge to the target status information, and send the feed rate of nutrient solution flow to the feeding operation subunit 522.

The feeding operation subunit 522 is used to feed the nutrient solution to the fermenting tank according to the received feed rate of nutrient solution flow.

Moreover, the control unit 520 may also comprise: a judgment subunit 523, which is used to receive the feed rate of nutrient solution flow from the flow feed rate determination subunit 521, and judge whether the feed rate of nutrient solution flow is within the preset range of flow feed rate; if yes, send the feed rate of nutrient solution flow to the feeding operation subunit 522; if no, send the preset target feed rate of nutrient solution flow to the feeding operation subunit 523.

Moreover, the control unit 520 may also comprise a target nutrient solution flow feed rate storage unit 524, which is used to store the target feed rate of nutrient solution flow and the flow feed rate range, and the judgment subunit 523 can obtain the target feed rate of nutrient solution flow and flow feed rate range from the target nutrient solution flow feed rate storage unit 524.

The target nutrient solution flow feed rate storage unit 524 can store the target feed rate of nutrient solution flow and the flow feed rate range that correspond to different application needs, and an operator can select a target nutrient feed rate and feed rate range suited to the current fermentation process according to different applications needs for judgment subunit 523 to conduct judgment, and can update the target nutrient feed rate and feed rate range at any time as needed.

## Claims

1. A method for controlling a fermentation process, the method comprising:

    A. collecting samples of biological cells with a flow cytometer from the fermenting tank and outputting a scatter plot comprising the current status information of the biological cells according to the biological cell samples, said status information comprising any one or any combination of cell concentration information, cell size information, cell form information, cell structure information and cell metabolism information;

    B. comparing said scatter plot of current status information of the biological cells with the scatter plot of the preset target status information to obtain the difference status between the current status information of the biological cells and the target status information, whereby said obtaining the difference status comprises:

        determining the center of gravity of the scatter plot of the current status information of the biological cells and the center of gravity of the scatter plot of said preset target status information;

        determining the vector characterizing said difference status, wherein the vector starts from the center of gravity of the scatter plot of the current status information of the biological cells, and points to and ends at the center of gravity of the scatter plot of said preset target status information;

    C. controlling the feed rate of nutrient solution flow to said fermenting tank according to said obtained difference status.

2. The method as claimed in claim 1, **characterized in that** said step C comprises:

converting the vector characterizing said difference status to a scalar;
determining the feed rate of nutrient solution flow that minimizes the scalar according to the converted scalar, and feeding the nutrient solution to said fermenting tank at the determined feed rate of nutrient solution flow.

3. The method as claimed in claim 1, **characterized in that** said step C comprises: determining the feed rate of nutrient solution flow according to the obtained difference status by using the preset flow feed rate determination strategy, and feeding the nutrient solution to said fermenting tank at the determined feed rate of the nutrient solution flow;
herein, said flow feed rate determination strategy causes the status information of the biological cells in said fermenting tank to converge to said target status information.

4. The method as claimed in claim 3, **characterized in that** said flow feed rate determination strategy includes proportional-integral-derivative (PID) strategy, model predictive control strategy, fuzzy control strategy and neural network control strategy.

5. The method as claimed in claim 2 or 3, **characterized in that** before feeding the nutrient solution to said fermenting tank at the determined feed rate of the nutrient solution flow, the method further comprises: judging whether said determined feed rate of nutrient solution flow is within the range of the preset flow feed rate; if yes, proceeding to said step of feeding the nutrient solution to said fermenting tank at the determined feed rate of nutrient solution flow; and if no, feeding the nutrient solution to said fermenting tank at the preset target feed rate of nutrient solution flow.

6. The method as claimed in claim 1, **characterized in that** the method comprises proceeding to said step A after performing said step C.

7. A device for controlling the fermentation process, comprising a flow cytometer, a comparison unit and a control unit;
said flow cytometer comprising: a collection subunit, a flow chamber, a laser generator and a detector;
said collection subunit is used to collect biological cell samples from the fermenting tank and inject the biological cell samples into the center of the sheath fluid in a flow chamber;
said flow chamber is used to allow the biological cells in said samples to align in a single line and form a cell bundle under the restriction of the sheath fluid;
said laser generator is used to generate a laser and excite said cell bundle to produce light information;
said detector is used to obtain the current status information of the biological cells from said light information according to the biological cell samples, said status information comprising any one or any combination of cell concentration information, cell size information, cell form information, cell structure information and cell metabolism information;
said comparison unit is used to compare said current status information of the biological cells with the preset target status information, obtain the difference status between the current status information of the biological cells and the preset target status information, and provide the difference status to said control unit, whereby said obtaining the difference status comprises:

determining the center of gravity of the scatter plot of the current status information of the biological cells and the center of gravity of the scatter plot of said preset target status information;
determining the vector characterizing said difference status, wherein the vector starts from the center of gravity of the scatter plot of the current status information of the biological cells, and points to and ends at the center of gravity of the scatter plot of said preset target status information;
said control unit is used to control the feed rate of nutrient solution flow to said fermenting tank according to said difference status.

8. The device as claimed in claim 7, **characterized in that** the device further comprises: a target status information storage unit for storing the target status information;
said comparison unit is also used to obtain said target status information from said target status information storage unit.

9. The device as claimed in claim 7, **characterized in that** said control unit comprises: a flow feed rate determination subunit and a feeding operation subunit; said flow feed rate determination subunit is used to determine the feed rate of nutrient solution flow based on said difference status by using the preset flow feed rate determination strategy which causes the status information of the biological cells in said fermenting tank to converge to said target status information, and to send the feed rate of nutrient solution flow to said feeding operation subunit; said feeding operation subunit is used to feed the nutrient solution to said fermenting tank according to the received feed rate of nutrient solution flow.

10. The device as claimed in claim 9, **characterized in that** said control unit further comprises: a judgment subunit, which is used to receive the feed rate of nutrient solution flow sent from said flow feed rate determination subunit, and judge whether the feed rate of nutrient solution flow is within the preset flow feed rate range; if yes, send said target feed rate of nutrient solution flow to said feeding operation subunit; if no, send the preset target flow feed rate of nutrient solution flow to said feeding operation subunit.

## Patentansprüche

**1.** Verfahren zum Steuern eines Fermentationsprozesses, wobei das Verfahren umfasst:

A. Gewinnen von Proben biologischer Zellen mit einem Durchflusszytometer aus dem Fermentierungstank und Ausgeben eines Streudiagramms, das die aktuellen Statusinformationen der biologischen Zellen gemäß den biologischen Zellproben umfasst, wobei die Statusinformationen eine beliebige oder eine beliebige Kombination von Zellkonzentrationsinformationen, Zellgrößeninformationen, Zellforminformationen, Zellstrukturinformationen und Zellmetabolismusinformationen umfasst;
B. Vergleichen des Streudiagramms der aktuellen Statusinformationen der biologischen Zellen mit dem Streudiagramm der vorgewählten Zielstatusinformationen, um den Differenzstatus zwischen den aktuellen Statusinformationen der biologischen Zellen und den Zielstatusinformationen zu erhalten, wobei das Erhalten des Differenzstatus umfasst:

Bestimmen des Schwerpunkts des Streudiagramms der aktuellen Statusinformationen der biologischen Zellen und des Schwerpunkts des Streudiagramms der vorgewählten Zielstatusinformationen;
Bestimmen des Vektors, der den Differenzstatus charakterisiert, wobei der Vektor von dem Schwerpunkt des Streudiagramms der aktuellen Statusinformationen der biologischen Zellen ausgeht und auf den Schwerpunkt des Streudiagramms der vorgewählten Zielstatusinformationen weist und dort endet;

C. Steuern der Zufuhrrate des Nährstofflösungsflusses zu dem Fermentierungstank gemäß dem erhaltenen Differenzstatus.

**2.** Verfahren nach Anspruch 1, **dadurch gekennzeichnet, dass** Schritt C umfasst:

Umwandeln des Vektors, der den Differenzstatus charakterisiert, in einen Skalar;
Bestimmen der Zufuhrrate des Nährstoffratenflusses, der gemäß dem umgewandelten Skalar den Skalar minimiert, und
Zuführen der Nährstofflösung zu dem Fermentierungstank mit der bestimmten Zufuhrrate des Nährstofflösungsflusses.

**3.** Verfahren nach Anspruch 1, **dadurch gekennzeichnet, dass** Schritt C umfasst: Bestimmen der Zufuhrrate des Nährstofflösungsflusses gemäß dem erhaltenen Differenzstatus durch Verwenden der Bestimmungsstrategie der vorgewählten Flusszufuhrrate und Zuführen der Nährstofflösung zu dem Fermentierungstank mit der bestimmten Zufuhrrate des Nährstofflösungsflusses;
wobei die Bestimmungsstrategie der Flusszufuhrrate hier bewirkt, dass die Statusinformationen der biologischen Zellen in dem Fermentierungstank zu den Zielstatusinformationen konvergieren.

**4.** Verfahren nach Anspruch 3, **dadurch gekennzeichnet, dass** die Bestimmungsstrategie der Flusszufuhrrate proportional-integral-derivative (PID)-Strategie, modellprädiktive Steuerungsstrategie, Fuzzy-Steuerungstrategie und neuronale Netzwerksteuerungsstrategie einschließt.

**5.** Verfahren nach Anspruch 2 oder 3, **dadurch gekennzeichnet, dass** das Verfahren vor dem Zuführen der Nährstofflösung zu dem Fermentierungstank mit der bestimmten Zufuhrrate des Nährstofflösungsflusses ferner umfasst: Beurteilen, ob die bestimmte Zufuhrrate des Nährstofflösungsflusses innerhalb des Bereichs der vorgewählten Flusszufuhrrate liegt; falls ja, Fortsetzen mit dem Schritt des Zuführens der Nährstofflösung zu dem Fermentierungstank mit der bestimmten Zufuhrrate des Nährstofflösungsflusses; und falls nein, Zuführen der Nährstofflösung zu dem Fermentierungstank mit der vorgewählten Zielzufuhrrate des Nährstofflösungsflusses.

**6.** Verfahren nach Anspruch 1, **dadurch gekennzeichnet, dass** das Verfahren Fortsetzen mit dem Schritt A nach Durchführen von Schritt C umfasst.

**7.** Vorrichtung zum Steuern des Fermentationsprozesses, umfassend ein Durchflusszytometer, eine Vergleichseinheit und eine Steuereinheit;
wobei das Durchflusszytometer umfasst: eine Gewinnungsuntereinheit, eine Durchflusskammer, einen Lasergenerator und einen Detektor;
die Gewinnungsuntereinheit zum Gewinnen von biologischen Zellproben aus dem Fermentierungstank und zum Injizieren der biologischen Zellproben in die Mitte des Hüllfluids in einer Durchflusskammer verwendet wird;

die Durchflusskammer verwendet wird, um den biologischen Zellen in den Proben das Ausrichten in einer Einzellinie und Bilden eines Zellbündels unter der Einengung durch das Hüllfluid zu bilden;
der Lasergenerator verwendet wird, um einen Laser zu generieren und das Zellbündel anzuregen, um Lichtinformationen zu produzieren;
wobei der Detektor verwendet wird, um die aktuellen Statusinformationen der biologischen Zellen aus den Lichtinformationen gemäß den biologischen Zellproben zu erhalten, wobei die Statusinformationen eine beliebige oder eine beliebige Kombination von Zellkonzentrationsinformationen, Zellgrößeninformationen, Zellforminformationen, Zellstrukturinformationen und Zellmetabolismusinformationen umfassen;
die Vergleichseinheit verwendet wird, um die aktuellen Statusinformationen der biologischen Zellen mit den vorgewählten Zielstatusinformationen zu vergleichen, den Differenzstatus zwischen den aktuellen Statusinformationen der biologischen Zellen und den vorgewählten Zielstatusinformationen zu erhalten und der Steuereinheit den Differenzstatus bereitzustellen, wobei das Erhalten des Differenzstatus umfasst:

Bestimmen des Schwerpunkts des Streudiagramms der aktuellen Statusinformationen der biologischen Zellen und des Schwerpunkts des Streudiagramms der vorgewählten Zielstatusinformationen;
Bestimmen des Vektors, der den Differenzstatus charakterisiert, wobei der Vektor von dem Schwerpunkt des Streudiagramms der aktuellen Statusinformationen der biologischen Zellen ausgeht und auf den Schwerpunkt des Streudiagramms der vorgewählten Zielstatusinformationen weist und dort endet;
wobei die Steuereinheit zum Steuern der Zufuhrrate des Nährstofflösungsflusses zu dem Fermentierungstank gemäß dem Differenzstatus verwendet wird.

8. Vorrichtung nach Anspruch 7, **dadurch gekennzeichnet, dass** die Vorrichtung ferner umfasst: eine Speichereinheit für die Zielstatusinformationen, um die Zielstatusinformationen zu speichern;
wobei die Vergleichseinheit auch verwendet wird, um die Zielstatusinformationen aus der Speichereinheit für die Zielstatusinformationen zu erhalten.

9. Vorrichtung nach Anspruch 7, **dadurch gekennzeichnet, dass** die Steuereinheit umfasst: eine Bestimmungsuntereinheit der Flusszufuhrrate und eine Zuführungsuntereinheit;
wobei die Bestimmungsuntereinheit der Flusszuruhrrate verwendet wird, um die Zufuhrrate des Nährstofflösungsflusses basierend auf dem Differenzstatus zu bestimmen, indem die Bestimmungsstrategie der vorgewählten Flusszufuhrrate verwendet wird, die bewirkt, dass die Statusinformationen der biologischen Zellen in dem Fermentierungstank zu den Zielstatusinformationen konvergieren, und die Zufuhrrate des Nährstofflösungsflusses zu der Zuführungsuntereinheit gesendet wird;
wobei die Zuführungsuntereinheit verwendet wird, um dem Fermentierungstank gemäß der empfangenen Zufuhrrate des Nährstofflösungsflusses die Nährstofflösung zuzuführen.

10. Vorrichtung nach Anspruch 9, **dadurch gekennzeichnet, dass** die Steuereinheit ferner umfasst: eine Beurteilungsuntereinheit, die verwendet wird, um die Zufuhrrate des Nährstofflösungsflusses zu empfangen, die von der Bestimmungsuntereinheit der Flusszufuhrrate gesendet wurde, und zu beurteilen, ob die Zufuhrrate des Nährstofflösungsflusses innerhalb des vorgewählten Flusszufuhrratenbereichs liegt; wobei, falls ja, die Zielzufuhrrate des Nährstofflösungsflusses zu der Zuführungsuntereinheit gesendet wird; und falls nein, die vorgewählte Zielflusszufuhrrate des Nährstofflösungsflusses zu der Zuführungsuntereinheit gesendet wird.

## Revendications

1. Procédé pour maîtriser un processus de fermentation, le procédé comprenant :

A. recueillir des échantillons de cellules biologiques par un cytomètre en flux d'une cuve de fermentation et sortir un graphique de diffusion, comprenant l'information d'état en cours des cellules biologiques, conformément aux échantillons de cellule biologique, l'information d'état comprenant l'une quelconque ou n'importe quelle combinaison d'une information de concentration de cellule, d'une information de dimension de cellule, d'une information de forme de cellule, d'une information de structure de cellule et d'une information de métabolisme de cellule ;
B. comparer le graphique de diffusion de l'information d'état en cours des cellules biologiques au graphique de diffusion de l'information d'état cible fixée à l'avance pour obtenir l'état de différence entre l'information d'état en cours des cellules biologiques et l'information d'état cible, dans lequel obtenir l'état de différence comprend :

déterminer le centre de gravité du graphique de diffusion de l'information d'état en cours des cellules biologiques et le centre de gravité du graphique de diffusion de l'information d'état cible fixé à l'avance ;
déterminer le vecteur caractérisant l'état de différence, le vecteur partant du centre de gravité du graphique de diffusion de l'information d'état en cours des cellules biologiques et pointant vers le centre de gravité du graphique de diffusion de l'information d'état cible fixé à l'avance et s'y terminant ;
C régler le débit d'alimentation en courant de solution nutritive de la cuve de fermentation en fonction de l'état de différence obtenu.

**2.** Procédé suivant la revendication 1, **caractérisé en ce que** le stade C comprend :

transformer le vecteur caractérisant l'état de différence en un scalaire ;
déterminer le débit du courant de solution nutritive qui minimise le scalaire selon le scalaire transformé et
alimenter la cuve de fermentation en la solution nutritive au débit déterminé de courant de solution nutritive.

**3.** Procédé suivant la revendication 1, **caractérisé en ce que** le stade C comprend : déterminer le débit de courant de solution nutritive, conformément à l'état de différence obtenu, en utilisant la stratégie de détermination du débit de courant fixé à l'avance et alimenter en la solution nutritive la cuve de fermentation au débit déterminé du courant de solution nutritive ;
dans lequel la stratégie de détermination du débit de courant fait que l'information d'état des cellules biologiques dans la cuve de fermentation converge sur l'information d'état cible.

**4.** Procédé suivant la revendication 3, **caractérisé en ce que** la stratégie de détermination du débit du courant comprend une stratégie proportionnelle-intégrale-dérivée (PID), une stratégie de commande prédictive de modèle, une stratégie de commande floue et une stratégie de commande de réseau neuronal.

**5.** Procédé suivant la revendication 2 ou 3, **caractérisé en ce qu'**avant d'alimenter la cuve de fermentation en la solution nutritive, au débit déterminé du courant de solution nutritive, le procédé comprend en outre : juger si le débit déterminé de courant de solution nutritive est dans la plage du débit de courant fixée à l'avance ; si oui, procéder au stade d'alimentation de la cuve de fermentation en la solution nutritive, au débit déterminé de courant de solution nutritive et si non, alimenter la cuve de fermentation en la solution nutritive, au débit cible fixé à l'avance de courant de solution nutritive.

**6.** Procédé suivant la revendication 1, **caractérisé en ce que** le procédé comprend, procéder au stade A après avoir effectué le stade C.

**7.** Dispositif pour maîtriser le processus de fermentation, comprenant un cytomètre à flux, une unité de comparaison et une unité de commande ;
le cytomètre à flux comprenant : une sous-unité de recueil, une chambre d'écoulement, un générateur laser et un détecteur ;
la sous-unité de recueil est utilisée pour recueillir des échantillons de cellule biologique de la cuve de fermentation et pour injecter les échantillons de cellule biologique dans le centre du fluide de gaine dans une chambre d'écoulement ;
la chambre d'écoulement est utilisée pour permettre aux cellules biologiques de l'échantillon de s'aligner suivant une ligne unique et de former un faisceau de cellule sous la restriction du fluide de gaine ;
le générateur laser est utilisé pour produire un laser et exciter le faisceau de cellule pour produire une information lumineuse ;
le détecteur est utilisé pour obtenir les informations d'état en cours des cellules biologiques à partir de l'information lumineuse, conformément aux échantillons de cellule biologique, l'information d'état comprenant n'importe laquelle ou n'importe quelle combinaison d'une information de concentration de cellule, d'une information de dimension de cellule, d'une information de forme de cellule, d'une information de structure de cellule et d'une information de métabolisme de cellule ;
l'unité de comparaison est utilisée pour comparer l'information d'état en cours des cellules biologiques à l'information d'état cible fixée à l'avance, pour obtenir l'état de différence entre l'information d'état en cours des cellules biologiques à l'information d'état cible fixée à l'avance et pour fournir l'état de différence à l'unité de commande, obtenir l'état de différence comprenant :

déterminer le centre de gravité du graphique de diffusion de l'information d'état en cours des cellules biologiques et le centre de gravité du graphique de diffusion de l'information d'état cible fixée à l'avance ;
déterminer le vecteur caractérisant l'état de différence, le vecteur partant du centre de gravité du graphique de diffusion de l'information d'état en cours des cellules biologiques et pointant vers le centre de gravité du graphique de diffusion de l'information d'état cible fixée à l'avance et s'y terminant ;
l'unité de commande est utilisée pour commander le débit d'alimentation de la cuve de fermentation en courant de solution nutritive, en fonction de l'état de différence.

**8.** Dispositif suivant la revendication 7, **caractérisé en ce que** le dispositif comprend en outre : une unité de mémorisation de l'information d'état cible, pour mémoriser l'information d'état cible ;
l'unité de comparaison est utilisée aussi pour obtenir l'information d'état cible à partir de l'unité de mémorisation de l'information d'état cible.

**9.** Dispositif suivant la revendication 7, **caractérisé en ce que** l'unité de commande comprend : une sous-unité de détermination du débit de courant et une sous-unité de fonctionnement de l'alimentation ;
la sous-unité de détermination du débit de courant est utilisée pour déterminer le débit de courant de solution nutritive sur la base de l'état de différence, en utilisant la stratégie de détermination de débit du courant fixé à l'avance, qui fait que l'information d'état des cellules biologiques dans la cuve de fermentation converge sur l'information d'état cible et, pour envoyer le débit de courant de solution nutritive à la sous-unité de fonctionnement de l'alimentation ;
la sous-unité de fonctionnement de l'alimentation est utilisée pour alimenter la cuve de fermentation en la solution nutritive, en fonction du débit reçu de courant de solution nutritive.

**10.** Dispositif suivant la revendication 9, **caractérisé en ce que** l'unité de commande comprend en outre : une sous-unité de jugement, qui est utilisée pour recevoir le débit de courant de solution nutritive envoyé de la sous-unité de détermination du débit de courant et pour juger si le débit de courant de la solution nutritive est dans la plage de débit de courant fixée à l'avance ; si oui, pour envoyer le débit cible de courant de solution nutritive à la sous-unité de fonctionnement d'alimentation ; si non, pour envoyer le débit de courant cible fixé à l'avance de courant de solution nutritive à la sous-unité de fonctionnement d'alimentation.

FIG 1

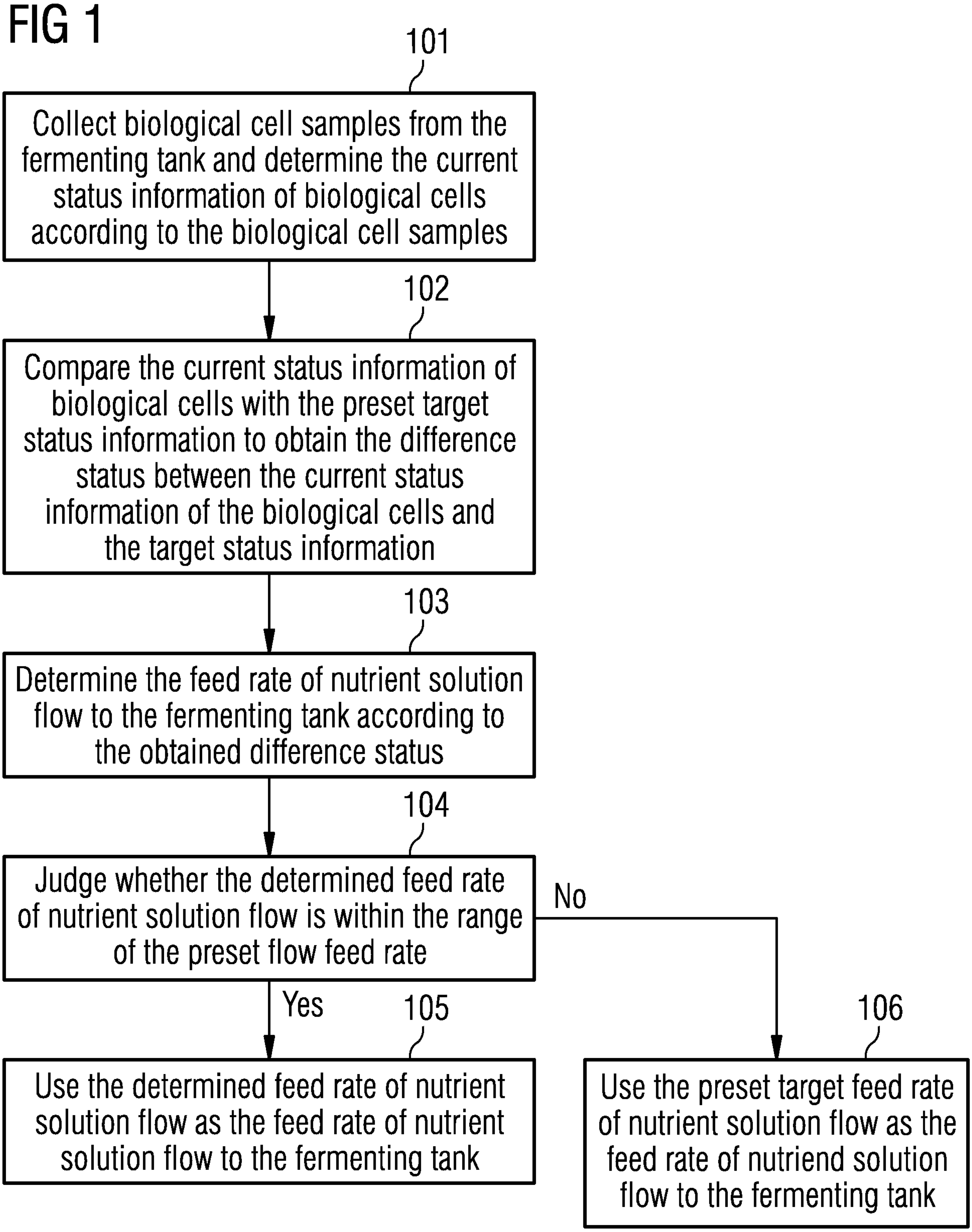
101
Collect biological cell samples from the fermenting tank and determine the current status information of biological cells according to the biological cell samples
102
Compare the current status information of biological cells with the preset target status information to obtain the difference status between the current status information of the biological cells and the target status information
103
Determine the feed rate of nutrient solution flow to the fermenting tank according to the obtained difference status
104
Judge whether the determined feed rate of nutrient solution flow is within the range of the preset flow feed rate
No
Yes
105
Use the determined feed rate of nutrient solution flow as the feed rate of nutrient solution flow to the fermenting tank
106
Use the preset target feed rate of nutrient solution flow as the feed rate of nutriend solution flow to the fermenting tank

FIG 2

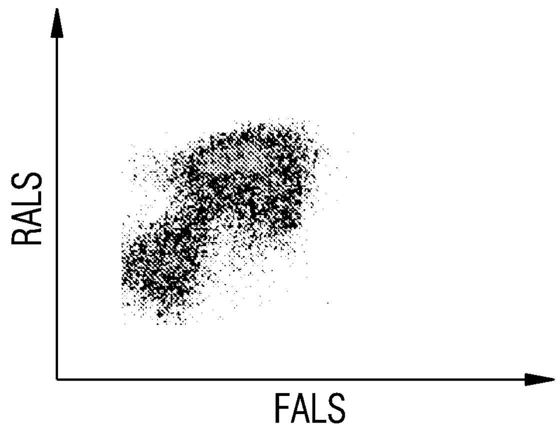


FIG 3

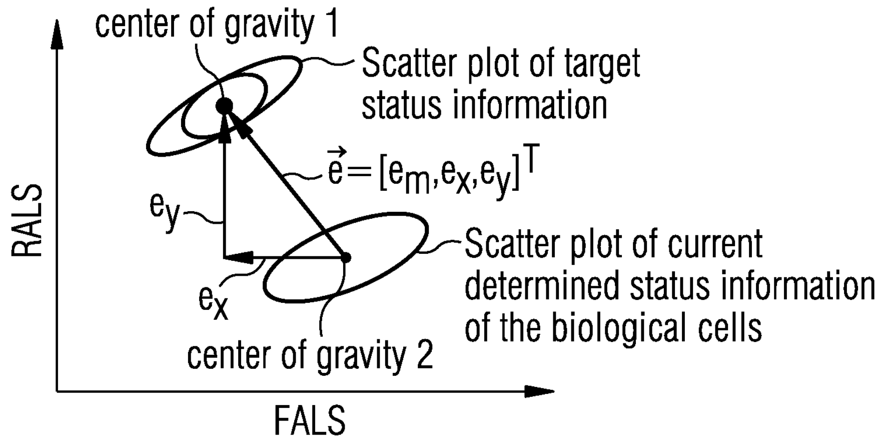


FIG 4

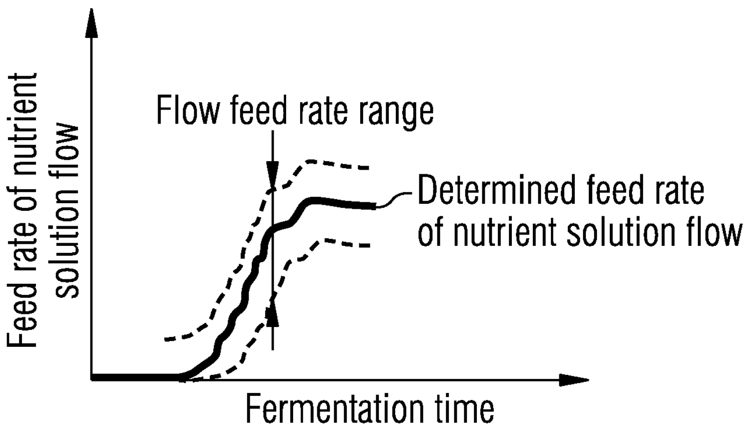

FIG 5

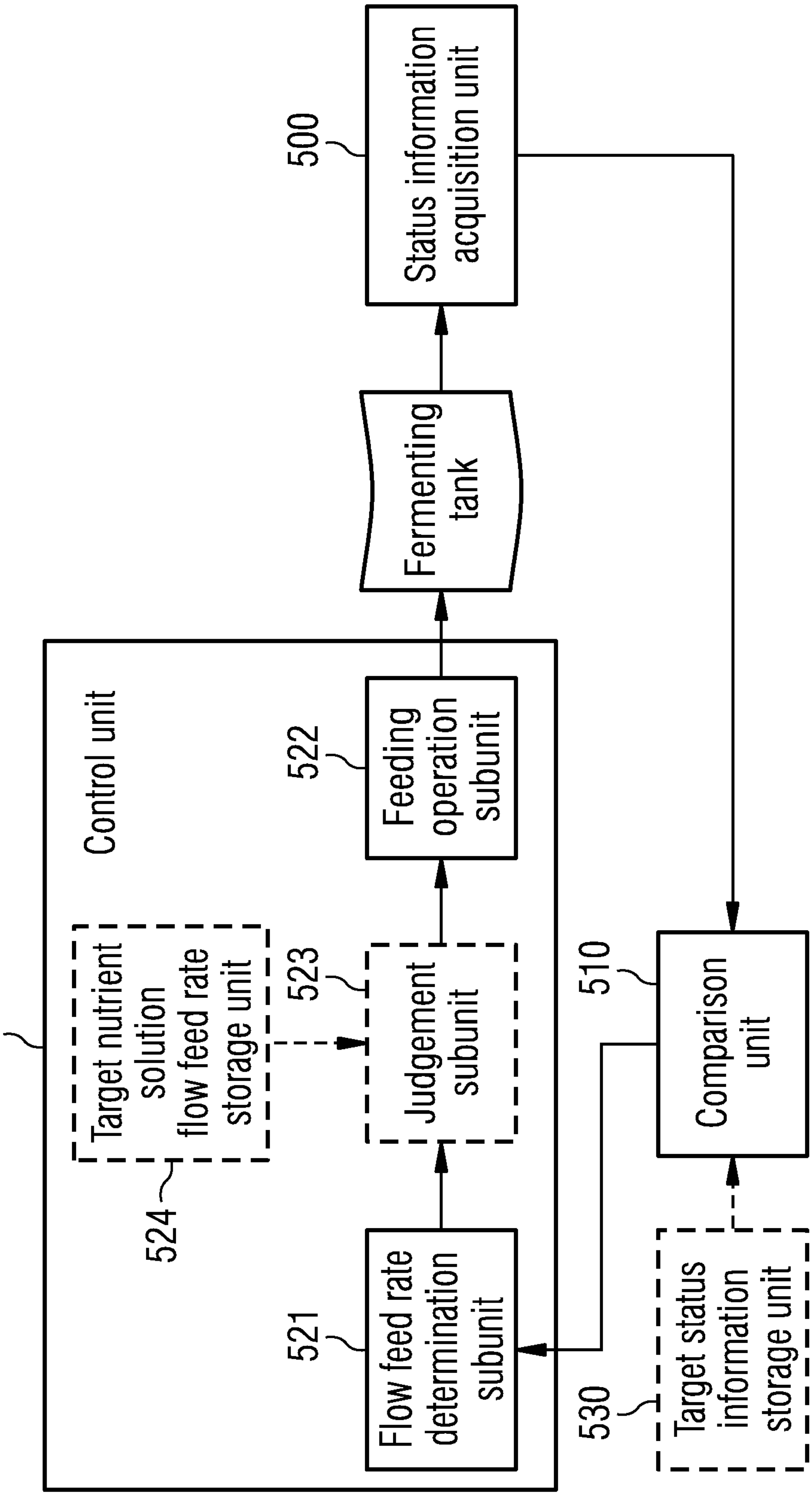
500
Status information acquisition unit
Fermenting tank
520
Control unit
522
Feeding operation subunit
523
Judgement subunit
Target nutrient solution flow feed rate storage unit
524
521
Flow feed rate determination subunit
510
Comparison unit
530
Target status information storage unit

FIG 6

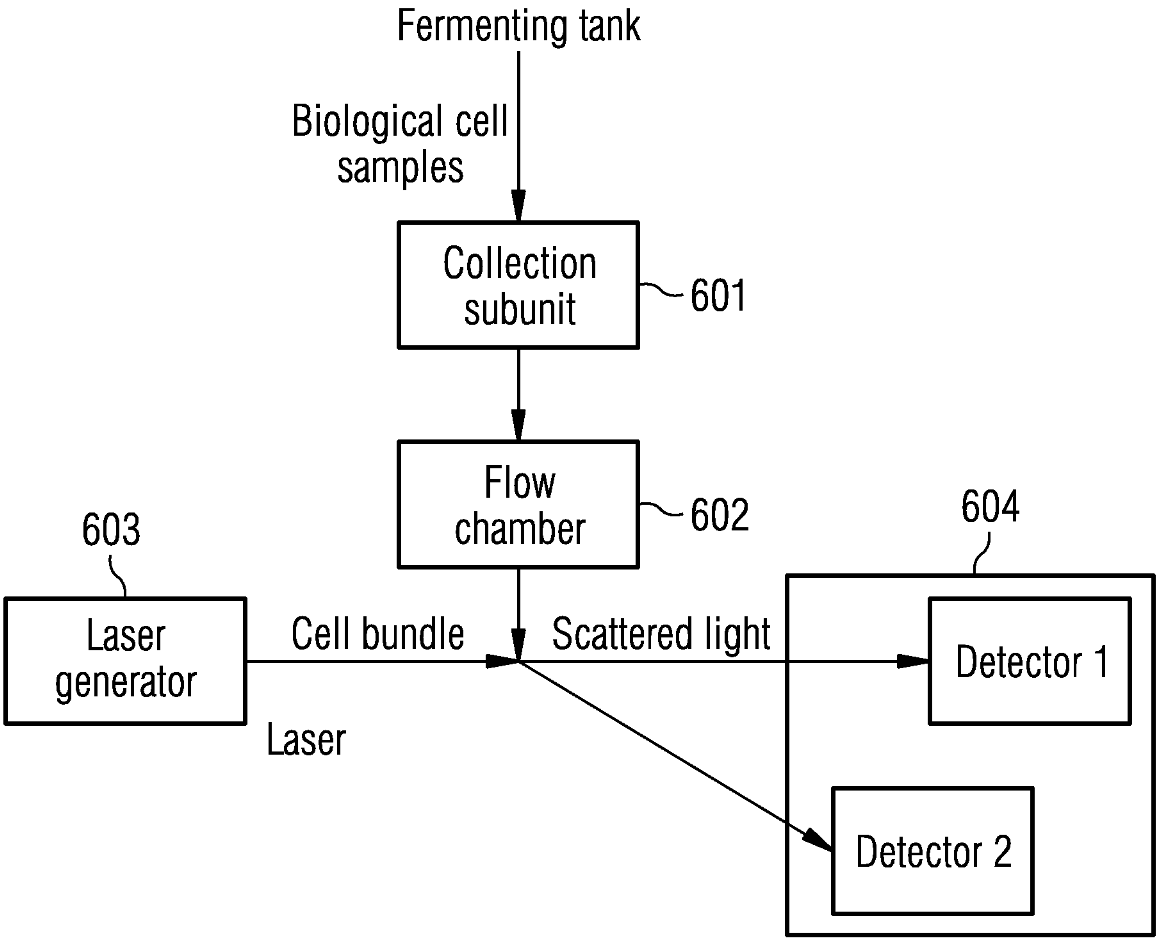
Fermenting tank
Biological cell samples
Collection subunit
601
Flow chamber
602
603
Laser generator
Cell bundle
Laser
Scattered light
604
Detector 1
Detector 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 02083836 A2


### Non-patent literature cited in the description


- **RUI ZHAO et al.** A flow injection flow cytometry system for on-line monitoring of bioreactors. *Biotechnology and Bioengineering,* 05 March 1999, vol. 62 (5), 609-617